(19) Europäisches Patentamt
European Patent Office
Office européen des brevets

(11) EP 2 378 291 A1

(12) EUROPEAN PATENT APPLICATION

(43) Date of publication:
**19.10.2011 Bulletin 2011/42**

(51) Int Cl.:
***G01N 33/94*** *(2006.01)* ***G01N 30/00*** *(2006.01)*

(21) Application number: **10003983.3**

(22) Date of filing: **15.04.2010**

(84) Designated Contracting States:
**AT BE BG CH CY CZ DE DK EE ES FI FR GB GR HR HU IE IS IT LI LT LU LV MC MK MT NL NO PL PT RO SE SI SK SM TR**
Designated Extension States:
**AL BA ME RS**

(71) Applicant: **UCB Pharma GmbH**
**40789 Monheim (DE)**

(72) Inventors:
- **Boekens, Hilmar**
  **40593 Düsseldorf (DE)**
- **Cawello, Willi**
  **40789 Monheim (DE)**

(74) Representative: **Dressen, Frank**
**UCB Pharma GmbH**
**Alfred-Nobel-Strasse 10**
**40789 Monheim (DE)**

# (54) Lacosamide detection assay

(57) The present application relates to a method of determining the anticonvulsant drug lacosamide in a body fluid sample, particularly in plasma, serum or saliva using a HPLC-UV method, and a lacosamide analogue as a standard. The present application also relates to a new method of determining the lacosamide content in a mammals body by measuring the lacosamide concentration or amount in the saliva.

EP 2 378 291 A1

## Description

Lacosamide ((R)-2-acetamido-N-benzyl-3-methoxypropionamide) is a D-serine derivative approved as an antiepileptic drug (AED) inter alia by the FDA and the EMEA for the adjunctive therapy of partial-onset seizures with and without secondary generalization.

Lacosamide was also proposed for use in treating status epilepticus including refractory status epilepticus (Wo 2007/144196) although it is not yet approved for this indication.

Lacosamide is available in oral form (tablets and/or syrup) and as an intravenous solution, which is particularly suitable for rapid administration in emergency cases such as e.g. repetitive seizures.

There is an increasing desire to have a tool to determine the blood concentration of lacosamide for several reasons: (1) to allow assessing causes for potential changes in drug responses, (2) to assess potential causes of clinical toxicity, (3) to assess compliance, (4) to ensure proper drug load, particularly in patients with breakthrough seizures, (5) to guide individual dosage adjustments, and (6) to accompany potentially important pharmacokinetic changes (from Patsalos et al, Epilepsia 49, 2008, 1239-1276). In addition to these "best practice" reasons, a rapid detection of lacosamide in emergency cases is also desirable. Patients suffering from uncontrolled repetitive seizures may benefit from a rapid bolus of intravenous lacosamide provided significant drug overdosing is avoided.

Therefore, a lacosamide detection assay should on one hand be as accurate, simple and robust as possible to allow the proper monitoring of routine therapy but should on the other hand be suited for standard hospital equipments to allow a rapid ad hoc performance in emergency situations.

Several methods have been described for the various anticonvulsants such as immunoassays, HPLC-MS, HPLC-UV, GC-MS, see Patsalos, supra. However, many of these assays are not suitable for the present purposes for several reasons. For example, mass spectrometry is not available in many hospitals and their use thus mostly limited to major laboratories. Immunoassays are difficult to develop for lacosamide due to the low size of the molecule (MW: 250 Da) and the very close structural similarity of lacosamide to its metabolites such as the major inactive desmethyl metabolite. Also, potential false negative results of immunoassays are to be avoided in emergency settings. Many other assays used to determine various other anticonvulsants are either not suited for lacosamide and/or are too slow and/or too complicated to be used in emergency situations.

A need therefore exists to develop a specific lacosamide detection assay which

(a) provides for a reliable determination of lacosamide concentrations in samples from patients
(b) is as quick as feasible
(c) should be easy and cheap to perform with the equipment which is usually available in hospitals
(d) is robust enough both in day-to-day practice, as well as in emergency situations
(e) optionally: is non-invasive

## Description of the Invention

It has been found, surprisingly, that a simple HPLC -UV assay fulfils all the criteria described further above, provided a suitable internal standard will be used.

Lacosamide has a molecular weight (MW) of 250 Da, whereas the pharmacologically inactive O-desmethyl main metabolite as well as other metabolites of lacosamide have MWs of about 236 Da and lower. It has been found by the present inventor that a suitable internal HPLC (reference) standard should have a molecular weight of between about 255 Da to 320 Da, preferably about 258 Da to 300 Da, more preferably between about 260 Da and 300 Da, even more preferably between 260 and 290 Da, between 260 and 280 Da, and most preferably between about 260 Da and about 278 Da, or between 262 Da and about 270 Da.

Moreover, the internal (reference) standard to be used in the present lacosamide detection assay should behave similar to lacosamide during the sample preparation for HPLC examination, and in the HPLC analysis as well. Particularly, the internal standard should have a similar recovery after protein precipitation, should be detectable at the same UV-wavelengtfl and should be eluted in HPLC close to the lacosamide itself, and should distinguish from other anticonvulsants in the before mentioned parameters thereby mimicking the analyte in the whole assay procedure. It has now been found that lacosamide homologues are particularly suited as internal standard (reference) in lacosamide detection assays.

Accordingly, one embodiment of the present disclosure relates to the use of lacosamide homologues, as further described herein, as a reference standard in lacosamide detection assays, preferably for determining the lacosamide concentration or amount in biological samples such as serum, plasma or saliva.

The term "lacosamide homologues" as used herein includes particularly compounds differing from the structure of lacosamide only by one or more additional groups selected from methyl, ethyl, propyl, methoxy, ethoxy, and halogen, and having a molecular weight of about 255 Da to 320 Da, and preferably between about 258 Da to 300 Da, more preferably between about 260 Da and 300 Da, even more preferably between 260 and 290 Da, or between 260 and

280 Da, and most preferably between about 260 Da and about 278 Da, or between 262 Da and about 270 Da.

Accordingly, one embodiment relates to a method of determining lacosamide in a sample of a mammal, preferably a human patient, wherein said method comprises the following subsequent steps:

a) adding to said sample a predefined amount of a compound having a MW of about 255-320 Da as an internal standard, said compound being preferably a lacosamide homologue
b) separating off at least 80%, preferably at least 90%, even more preferably at least 95% of the protein content from said sample,
c) subjecting the protein diminished sample of step b) to an HPLC analysis under appropriate conditions, and
d) determining the amount and/or concentration of lacosamide and said internal standard via a suitable detection mode, preferably by UV light, on said HPLC column, and
e) calculating the amount and/or concentration of lacosamide in the sample of the mammal.

In one embodiment, the internal standard of the present invention is a lacosamide homologue having the chemical formula I

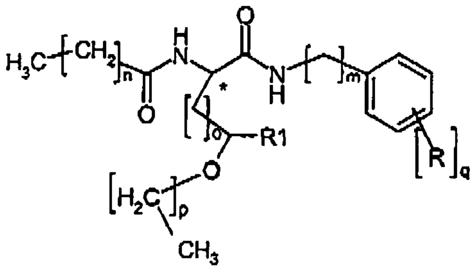

wherein
* indicates a chiral center;
each R is independently selected from methyl, methoxy, fluoro and chloro, R1 is hydrogen or methyl
m is 1 or 2,
n, o, and p are independently 0 or 1
q is 0, 1, or 2; and
the molecular weight of the internal standard is between about a molecular weight of about 255 Da to 320 Da, and preferably between about 258 Da to 300 Da, more preferably between about 260 Da and 300 Da, even more preferably between 260 and 290 Da, or between 260 and 280 Da, and most preferably between about 260 Da and about 278 Da, or between 262 Da and about 270 Da,
and wherein the internal standard may be in form of a pure enantiomer, diastereomer, or any mixture of enantiomers.

One embodiment of the present disclosure relates to a method of determining the lacosamide concentration in a sample of a mammal, preferably a body fluid sample derived from a mammal, said method comprising the following subsequent steps:

a) adding to said body fluid sample a predefined amount of an internal standard being a lacosamide homologue having the chemical formula I

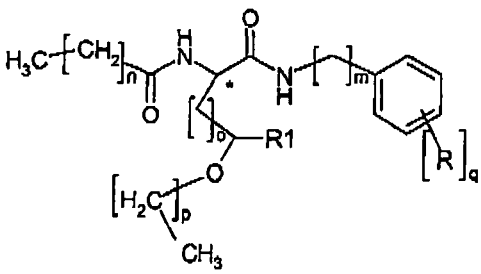

wherein
* indicates a chiral center;
each R is independently selected from methyl, methoxy, fluoro and chloro, R1 is hydrogen or methyl
m is 1 or 2,
n, o, and p are independently 0 or 1
q is 0, 1, or 2; and
the molecular weight of the internal standard is between about 258 and about 300 Da, preferably of 260-280 Da, and wherein the internal standard may be in form of a pure enantiomer, diastereomer or any mixture of enantiomers,
b) separating off at least about 90% (w/w) of the protein so as to provide a protein diminished sample,
c) analytically separating a pretermined volume of the protein diminished sample (e.g. the supernatant following centrifugation) of step b) on an HPLC column which has been previously calibrated with said internal standard of formula I.
d) determining the peakheights or peakareas of lacosamide and of the internal standard of formula I after the HPLC separation of step c), preferably via UV detection, and
e) calculating the amount and/or concentration of lacosamide in the body fluid sample using the comparison of the peakheights or peakareas of lacosamide with the respective value of said internal standard of formula I

In one preferred embodiment of the present invention, the internal standard has the formula II

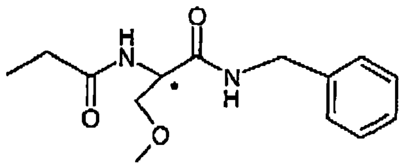
formula II

and may be used in form of a pure enantiomer, or any mixture of enantiomers.

Typically, the internal standard will be added to the sample prior to the protein separation step. In a typical example, 1-3 μg of internal standard will be added per each 100 μl of sample. Typically a concentration of lacosamide in the range of between about 0.2 and 20μ g/mL, more preferably between 0.5 and 15μg/ml, or 2 and 15μg/ml can be determined in the sample.

It is clear to one skilled in the art that once the lacosamide concentration in determined in a sample, usually the amount of lacosamide can also be calculated. Hence, in some instances the terms "lacosamide content" or lacosamide amount also include the lacosamide concentration, and vice versa.

The term "internal standard", "reference" or "reference standard" refers to a compound as further defined herein, which will be added to the sample some time prior to analytical (e.g. HPLC) analysis in a predetermined amount and which will be used as a reference to determine the amount of lacosamide in such sample. This can be done by comparing either the peakareas or, preferably, the peakheights of both the internal standard added and lacosamide in said sample, and by calculating the concentration of lacosamide according to the following formula:

Concentration of lacosamide in samples = [concentration of lacosamide in calibration sample/RFcalibration] x RFsample

The determination of the reference factor RF can be done during calibration of the HPLC system at any time prior to the presently disclosed assay by comparing the peakheights or peakareas of predetermined amounts (e.g. 10 μg/ml) of lacosamide and said internal standard and by calculating RF as follows: RF = peakheight lacosamide/ peakheight internal standard.

In one preferred embodiment of the presently disclosed method the sample is a body fluid sample, preferably plasma, serum, lymph fluid, or saliva. In one embodiment, the body fluid sample is blood, and the method further comprises the removal of at least 90%, preferably at least 95%, at least 96%, at least 97%, at least 98%, at least 99% or even more preferably substantially all of the blood cells by centrifugation or coagulation prior to step a).

Preferably the sample to be used in the method herein is derived from a human being, preferably from a patient suffering from epilepsy, pain, migraine, or subforms thereof, particularly from a patient suffering from an acute episode of epilepsy including e.g. uncontrolled seizures, status epilepticus, epilepsy with repeated partial seizures with and

without generalization, or with generalized seizures.

In one embodiment, the protein is separated off in step b) by precipitation and subsequent centrifugation or filtration to provide a supernatant, from which at least about 80%, at least 90%, preferably, at least about 92%, 93%, 94%, 95%, 96%, 97%, 98%, or even more than 99% of the protein from the original sample is removed.

The precipitation will be performed by adding a precipitating agent, which preferably comprises an agent selected from the group of alcohols, acids or organic solvents, and more preferably comprises methanol, ethanol, acetonitrile, acetone, trichloroacetic acid (TCA), perchloric acid, acidified methanol, acidified ethanol, acidified acetone, or mixtures thereof. In one specific embodiment, the precipitation agent is methanol. The volume ratio of sample to precipitating agent is preferably at least about 1: 1.5, more preferably at least about 1:2 or at least about 1:3. Preferably, the protein precipitation from the sample will be performed at room temperature. In one example methanol will be used as precipitating agent and will be added to the sample in a volume ratio of about 2.5:1 to 3:1 at room temperature.

In one embodiment of the method disclosed herein, in step b the precipitated protein may be removed from the protein diminished supernatant by filtration or centrifugation, preferably by centrifugation. Any centrifuge may be used according to this invention, wherein those centrifuges are preferred in which small vials such as e.g. Eppendorf tubes can be used. The protein may be centrifuged at about 3000 g or more, at about 5000g or more, preferably at about 10000 g or more, or even at about 15000 g or more.

Various HPLC columns including normal phase and reversed phase columns could be used in the method of the present disclosure. In one preferred embodiment the HPLC column is a reversed phase column, preferably comprising an organically substituted silane phase, more preferably a substituted dimethylsilane phase. Such columns are known to persons skilled in the art, and may comprise alkyl-, phenyl-, or nitrile- substituted silane, preferably dimethylsilane phases, wherein those HPLM colums are preferred which comprise an alkyldimethylsilane phase, wherein the alkyl chains each have between 2 and about 20 carbon atoms, more preferably between 5 and 20, or between 5 and 10 carbon atoms. A particularly preferred HPLC column is a C8-(octyl)-silica phased column, such as the commercially available column Zorbax® Eclipse XDB-C8 from the company Agilent Technologies.

Suitable sizes of the column may vary in length between about 18 and 300 mm, preferably between about 50 and about 150 mm, and may have a diameter of between about 2 and about 5 mm.

The mobile phase used in the HPLC separation varies depending on the choice of the column and the nature of the sample, and can be adapted by a person skilled in the art, accordingly.

If a reversed phase HPLC column will be used in the method as further disclosed herein, a polar eluent, preferably a mixture of an aqueous, slightly acidic buffer such as e.g. an alkali phosphate buffer, and an organic solvent, such as e.g. acetonitrile or methanol, may be used. The elution may be isocratic or as a gradient, wherein in the presently disclosed method preferably an isocratic elution will be performed.

In one preferred embodiment the elution from the reversed phase HPLC column will be performed using a mixture of an alkali phosphate buffer and acetonitrile at a pH of about 3-5; and preferably of about 4-5. In one example, a mixture of about 5-20 mM potassium or sodium phosphate, pH 4-5, with acetotrile in a ratio (v/v) of 7:3 to 9:1, preferably of about 8:2 to 9.1 will be used as eluent. The elution may be performed at a flux rate of 0,5-2 ml/min, preferably at about 0,8-1,8 ml/min, and even more preferably between about 1-1,5 ml/min.

HPLC parameters like the lengths of the column and the flow rate can be balanced between the desire

- to allow a reliable determination of lacosamide concentrations in samples which may also contain other anticonvulsants that must be separated from lacosamide, and
- to provide results as quick as feasible, particularly when the patient is in an emergency situation.

The detection of lacosamide in the present method will be typically performed using a UV detector at about 200-215 nm, preferably at about 205 nm. This allows for a quick and simple but robust and sensitive determination of the lacosamide content.

One embodiment of the present method relates to the determination of the concentration and/or amount and/or concentration of lacosamide in a patient's blood or body comprising a method comprising the subsequent steps of

aa) taking a sample from a patient's body, and removing at least 90%, preferably at least 95%, 96%, 97%, 98%, or 99% or more of the cells therein, if present,

- conducting the steps a) to e) as further described herein, and

f) calculating the concentration and/or amount of lacosamide in the patient's blood/plasma or body.

Once the concentration of lacosamide in the plasma of the mammal, preferably the human patient, has been

determined, it can be compared with the target concentration of lacosamide in a patient under treatment with lacosamide. Suitable therapeutic plasma concentrations range between about 0.5 and about 20 μglml, and preferably between about 2 and about 15, or between about 5 and 15 μg lacosamide/ml plasma, depending on the severity of the disease, the constitution of the patient, and time after actual lacosamide administration.

Based on the determination of the actual lacosamide concentration in the plasma and the comparison with the target concentration, either an individual dosing may be selected, or, alternatively, the general treatment and dosage schedule of the individual patient may be adapted such as to achieve higher or lower steady state concentrations of lacosamide. The latter can be done by adopting the respective daily dosages e.g. by adopting the dosage intervals of said patient or, preferably, by adapting the individual dosages administered.

One embodiment of the present invention relates to a method of administering to a patient in need thereof an individual dosage of lacosamide comprising the subsequent steps of

aa) taking a sample from a patient's body, and removing at least 90%, preferably at least 95%, 96%, 97%, 98%, or 99% or more of the cells therein, if present, conducting the steps

a) to e) as further described herein,
f) calculating the concentration and/or amount of lacosamide in the patient's blood or body, and either

g1) calculating the individual amount of lacosamide to be acutely administered to the patient based on the lacosamide concentration or amount already present in the patient's body or plasma, and/or
g2) adopting the dosage regime of said patient to increase or decrease the steady state concentration of lacosamide in said patient.

The lacosamide assay disclosed herein allows for a rapid, reliable, and robust determination of the lacosamide concentration. The time needed for the performance of the individual steps of the assay is given in table 1:

Table 1

| Step | Approximate time needed (min) |
|---|---|
| (a) addition of internal standard from stock solution | 1-2 |
| (b) protein precipitation and centrifugation | 5-12 min (depending on e.g. centrifuge used) |
| (c) HPLC-UV analysis | 10-14 min (at 1,5-1 ml/min, respectively) |
| (d) calculation of lacosamide concentration | 1-2 min |
| Total time needed | About 17-30 min |

One embodiment relates to kit, such as for use in emergency situations, comprising

i) an internal standard having the chemical formula I

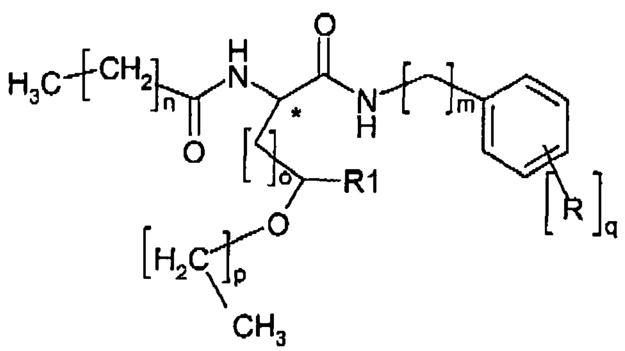

wherein
* indicates a chiral center;

each R is independently selected from methyl, methoxy, fluoro and chloro, R1 is hydrogen or methyl
m is 1 or 2,
n, o, and p are independently 0 or 1
q is 0, 1, or 2; and
the molecular weight of the internal standard is between about 258 and about 300 Da, preferably of 260-280 Da and wherein the internal standard may be in form of a pure enantiomer, diastereomer or any mixture of enantiomers,
ii) instructions for determing the lacosamide concentration in a patients body using the internal standard of formula I, and
iii) one or more dosing units of lacosamide.

The term "dosing unit of lacosamide" refers to a galenic unit or portion containing a predefined amount of lacosamide. Preferred dosing units of lacosamide contain between 50 mg and about 600 mg of lacosamide, while dosing units of 50 mg, 100 mg, 150 mg, 200 mg and 300 mg are preferred if taken twice a day. The dosing units may be formulated as tablets, syrup, IV solution, and the like. In one embodiment, the emergency kit of the present invention may comprise one or more dosing units in the form of an intravenous (IV) solution which is suitable for the rapid "bolus" injection in emergency cases, wherein the IV solution may preferably contain 100 mg, 150 mg, or 200 mg of lacosamide. In one embodiment, the emergency kit may contain one or more different dosing units of lacosamide, such as e.g. one or more vials containing e.g. 200 mg lacosamide intravenous solution, and one or more tablets, e.g. 50 mg, 100 mg, 150 mg or 200 mg tablets.
One preferred embodiment relates to a kit, such as an emergency kit, as described herein, wherein the internal standard has the formula

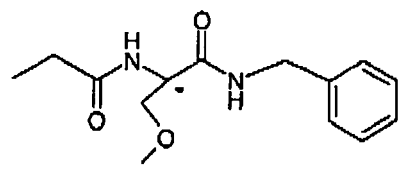

wherein the internal standard may be in form of a pure enantiomer, or any mixture of enantiomers.
One embodiment of the present disclosure relates to the use of saliva samples as a tool to assess the concentration of lacosamide in the body of a mammal, particularly a human being. It has been found, surprisingly, that there is a good correlation between the concentration of lacosamide in saliva and in the plasma of a mammal, particularly in humans. Preferably, saliva samples should be taken at least about 1 hour, preferably at least about 2 hours after an actual administration of lacosamide to said mammal to allow a steady state concentration to form, and to allow the proportional distribution of lacosamide into the saliva.
The method described herein provides for the first time a determination of lacosamide plasma levels by the measurement of the lacosamide concentrations in saliva samples, which has several advantages:

- the collection of saliva is easier and quicker than taking blood/plasma samples
- improved patient compliance since no invasive steps are needed to take blood/plasma
- the collection of saliva can be performed by the patient himself, and does normally not require professional healthcare support

Accordingly, one embodiment of the current invention relates to the use of a saliva sample to assess the concentration of lacosamide in the body of a mammal, particularly in the plasma of a patient This can be done based on the recognition that the concentration of lacosamide in saliva is about 1.17 times the concentration in plasma. Based on the measurement of lacosamide in saliva, the concentration in plasma can be easily assessed using the equation C plasma= C saliva x about 0.855 (see Example 3 in the experimental part). The determination of lacosamide in saliva should be done in the steady state, which is usually reached after about one hour to two hours after the last drug administration to the mammal/patient from which the saliva sample is to be taken.
One embodiment of the present invention relates to a method of determining the concentration of lacosamide in the body of a mammal, particularly of the lacosamide concentration in the blood/plasma/serum of a patient by the steps of

(1) determining the concentration of lacosamide in the saliva sample of a mammal, particularly of a human patient, and
(2) calculating the concentration of lacosamide in the plasma of the mammal, particularly the human patient

One embodiment of the present disclosure relates to a method of determining the concentration of lacosamide in the body of a mammal, comprising the subsequent steps of
pre (1) taking a saliva sample from a mammal, e.g. from a human patient,

(1) determining the concentration of lacosamide in the saliva sample of a mammal, particularly of a human patient,
(2) calculating the concentration of lacosamide in the plasma of the mammal, particularly the human patient, and optionally either
(3a) calculating the individual amount of Lacosamide to be acutely administered to the patient based on the lacosamide concentration or amount already present in the patient's body, pharmacokinetic distribution volume, or plasma, and/or
(3b) adapting the dosage regime of said patient to increase or decrease the steady state concentration of lacosamide in said patient.

One embodiment of the present disclosure relates to a method of determining the lacosamide concentration in the saliva sample of a mammal, particularly of a human patient, and thereafter calculating the concentration or amount of lacosamide in the body, pharmacokinetic distribution volume, or plasma of said mammal, particularly the human patient, and optionally either

(1) calculating the individual amount of lacosamide to be acutely administered to the patient based on the lacosamide concentration or amount already present in the patient's body, pharmacokinetic distribution volume, or plasma, and/or
(2) determining the otimized dosage regime of said patient, optionally followed by increasing or decreasing the steady state concentration of lacosamide in said patient.

The amount and/or concentration of lacosamide in the saliva sample may be determined by several methods, including for example immunoassays using lacosamide-specific antibodies, or fragments thereof, or may be done via GC, GC-MS, GC-MS/MS, LC-MS, LC-MS/MS or HPLC-MS or HPLC-UV methods, such as for example using the method disclosed herein. In a preferred embodiment, the saliva samples are measured via a HPLC-UV method as further disclosed in detail in this specification.
In one embodiment of the present invention the saliva will be treated to reduce its viscosity prior to conducting analytical steps. This may be done, e.g. by adding a compound with a free SH-group such as e.g. cystein or, preferably, N-acetyl cystein. However, the addition of such compounds is less preferred in UV detection methods since it may interfere with the detection of lacosamide and the reference standard in the sample.
The invention is further described by the following, non-limiting items:

(1) Method of determining the lacosamide concentration in a body fluid sample derived from a mammal, said method comprising the following subsequent steps:

a) adding to said body fluid sample a predefined amount of an internal standard being a lacosamide homologue having the chemical formula I

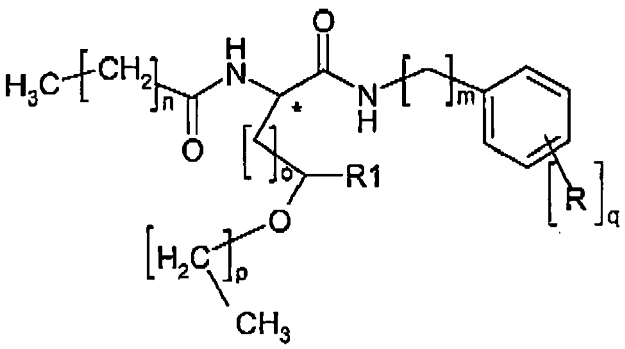

wherein
* indicates a chiral center;
each R is independently selected from methyl, methoxy, fluoro and chloro,

$R^1$ is hydrogen or methyl
m is 1 or 2,
n, o, and p are independently 0 or 1
q is 0, 1, or 2; and
having a molecular weight of between about 255 Da to 320 Da, preferably about 258 Da to 300 Da, more preferably between about 260 Da and 300 Da, even more preferably between 260 and 290 Da, between 260 and 280 Da, and most preferably between about 260 Da and about 278 Da, or between 262 Da and about 270 Da, and wherein the internal standard may be in form of a pure enantiomer, diastereomer or any mixture of enantiomers,
b) separating off at least about 90% (w/w) of the protein so as to provide a protein diminished sample,
c) analytically separating a pretermined volume of the protein diminished sample (e.g. the supernatant following centrifugation) of step b) on an HPLC column which has been previously calibrated with said internal standard of formula I,
d) determining the peakheights or peakareas of lacosamide and of the internal standard of formula I after the HPLC separation of step c), and
e) calculating the amount and/or concentration of lacosamide in the body fluid sample using the comparison of the peakheights or peakareas of lacosamide with the respective value of said internal standard of formula I.

(2) The method of item 1, wherein the internal standard of formula I is the compound

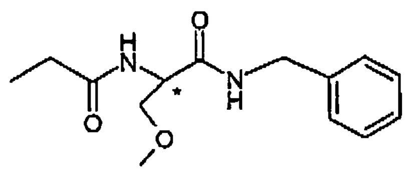

(3) The method of items 1 or 2, wherein the body fluid sample is blood, serum, plasma or saliva.
(4) The method of items 1 or 2, wherein the body fluid sample is blood, and wherein the method further comprises the removal of blood cells by centrifugation prior to step a)
(5) The method according to anyone of the previous items wherein the precipitating agent comprises an agent selected from the group of alcohols, acids or organic solvents
(6) The method of item 5, wherein the precipitating agent comprises methanol, ethanol, acetonitrile, acetone, trichloroacetic acid (TCA), perchloric acid, acidified methanol, acidified ethanol, acidified acetone, or mixtures thereof
(7) The method according to anyone of items 1-4, wherein the precipitation agent is methanol, and wherein the volume ratio of methanol, body fluid sample is at least about two
(8) The method according to anyone of the preceding items, wherein the precipitated protein in step b of item 1 is removed by centrifugation
(9) The method of anyone of the preceding items, wherein the HPLC column is a reversed phase column comprising an organically substituted silane surface
(10) The method of item 8, wherein the HPLC column comprises an alkyldimethylsilane surface wherein the alkyl chains each have between 5 and about 20 carbon atoms.
(11) The method of item 9, wherein the alkyl chains attached to the dimethylsilane surface have an average length of 5 to 10 carbon atoms
(12) The method according to anyone of the preceding items, wherein the detection of lacosamide during HPLC separation will be performed at about 200-215 nm
(13) The method according to anyone of the preceding items wherein the separation according to step c of item 1 will be performed using as the eluent a mixture of an alkali phosphate buffer and acetonitrile at a pH of about 3-5; preferably of about 4-5, and a at a flux rate of 0,8-1,5 ml/min.
(14) The method according to item 12, wherein an isocratic elution will be performed.
(15) Method of determining the concentration of lacosamide in a patients's blood comprising a method according to anyone of the preceding items, and subsequently calculating the blood level of lacosamide based on the lacosamide concentration in the body fluid sample.
(16) Kit, preferably for use in an emergency situation, comprising

(i) an internal standard having the chemical formula I

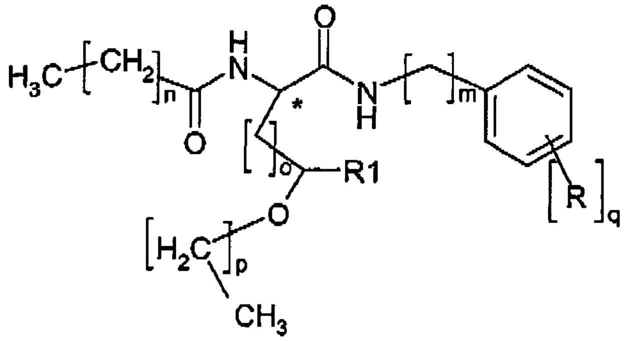

wherein
* indicates a chiral center;
each R is independently selected from methyl, methoxy, fluoro and chloro, R1 is hydrogen or methyl
m is 1 or 2,
n, o, and p are independently 0 or 1
q is 0, 1, or 2; and
the molecular weight of the internal standard is between about 255 Da to 320 Da, preferably about 258 Da to 300 Da, more preferably between about 260 Da and 300 Da, even more preferably between 260 and 290 Da, between 260 and 280 Da, and most preferably between about 260 Da and about 278 Da, or between 262 Da and about 270 Da,
and wherein the internal standard may be in form of a pure enantiomer, diastereomer or any mixture of enantiomers,
(ii) instructions for determing the lacosamide concentration in a patients body using the internal standard of formula I, and
(iii) one or more dosing units of lacosamide

(17) Kit of item 16, wherein the internal standard has the formula

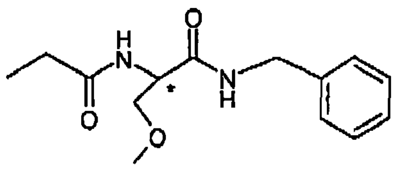

(18) A method of determining the concentration of lacosamide in the body of a mammal, particularly of the lacosamide concentration in the blood/plasme/serum of a patient by the steps of

(1) determining the concentration of lacosamide in a saliva sample of a mammal, particularly of a human patient, and
(2) calculating the concentration of lacosamide in the plasma of the mammal, particularly the human patient based on the lacosamide concentration in the saliva sample

(19) A method according to item 18 comprising the subsequent steps of
pre (1) taking a saliva sample from a mammal, e.g. from a human patient,

(1) determining the concentration of lacosamide in the saliva sample of a mammal, particularly of a human patient,
(2) calculating the concentration of lacosamide in the plasma of the mammal, particularly the human patient based on the lacosamide concentration in the saliva sample, and optionally either
(3a) calculating the individual amount of lacosamide to be acutely administered to the patient based on the lacosamide concentration or amount already present in the patient’s body or plasma, and/or
(3b) adopting the dosage regime of said patient to increase or decrease the steady state concentration of lacosamide in said patient.

(20) Use of lacosamide homologues, as further described herein, as a reference standard in lacosamide detection assays, preferably for determining the lacosamide concentration or amount in biological samples such as serum, plasma or saliva.

## Experimental Part

### Example 1:

#### 1 INSTRUMENTATION AND SOFTWARE

The following or equivalent instrumentation and software can be used. The instrumentation listed herein is thought as an example for illustrative purposes only and is in no way limiting.

#### 1.1 Chromatographic system / Detection

High performance liquid chromatography (HPLC) system Agilent

1100 with ChemStation chromatography software consisting of:

Wellplate Autosampler
Binary pump used in the isocratic mode
Variable wavelenth UV Detector (set at 205 nm)
Analytical Column: Zorbax® Eclipse XDB-C8, 150 x 4.6 mm

#### 2 BLANK MATRIX AND SAMPLES

#### 2.1 Blank matrix

For the preparation of calibration and quality control samples blank human plasma with heparin as anticoagulant is used.

#### 2.2 Calibration, quality control and instrument control samples

The assay is calibrated by one point calibration at 10 μg/mL, 3 replicate samples.

#### 2.3 Study samples

Study plasma samples can be stored at -12°C to -30°C until they are thawed at room temperature on the day of analysis or can be introduced in the assay immediately after drawing.

#### 3 SOLUTIONS AND REAGENTS

#### 3.1 Stock and working solutions of the standard compounds

| **Stock and working solutions of LCM** | |
|---|---|
| Stock solution lacosamide (c = 1000 μg/mL): | 10 mg of the respective analyte is dissolved in 10 mL of water. |

| **Stock solution of the internal standard** | |
|---|---|
| Stock solution (c = 2000 μg/mL): | 10 mg int.standard is dissolved in 5 mL of water. |

**Working solution of the internal standard**

| | |
|---|---|
| Working solution (c = 200 μg/mL): | 1mL of the stock solution is filled up with water to 10 mL. |

**Working solution for calibration**

| | |
|---|---|
| Working solution (LCM: c = 100 μg/mL; Int. Std.: c= 200 μg/mL): | 1 mL of both stock solutions are filled up with water to 10 mL |

All stock and working solutions are stored in glass vessels in a refrigerator at ca. 5°C.

**4 SAMPLE PREPARATION**

100 μL of plasma are transferred into Eppendorf tubes.
Calibration samples are added 10 μL working solution.
Study samples are added 10 μL of working solution int standard
After mixing 300 μL of methanol were added, the mixture is vortexed again and centrifuged
10 minutes at room temperature. 70 μL of the supernatant were transferred into a autosamplervial.
15 μL were injected into the HPLC-system.

**5 CHROMATOGRAPHY AND DETECTION**

**HPLC parameters**

| | |
|---|---|
| Column temperature: | ambient |
| Mobile Phase: | 10 mM potassium phosphate, pH 4.5/ACN; 81,5:18,5 (V/V) |
| Flow rate: | 1 mL/min |
| Injection volume: | 15 μL |
| Runtime : | 12 min |
| Elution : | isocratic |
| UV: | 205 nm |
| Retention time: LCM: | 6.8 min |
| Retention time: int. Std | 10.6 min |

**6 CALCULATION OF RESULTS**

Because of some matrix interferences, calculations of results were done preferably using peakheights.
For calibration the mean response factor for the peakheight ratios at 10 μg/mL was calculated:

RF = peakheight lacosamide/ peakheight int. Std.
Concentration in samples were calculated: 10/RFcalibration*RFsample

**7 METHOD SPECIFICATION**

Recovery of LCM after protein precipitation: > 90%(plasma); > 65%(saliva) Linearity of calibration curves, R > 0.999
Intra- and Interassay accuraty in the calibration range: within the required range of 85-115%
Intra- and Interassay precision in the calibration range: within the required range of <15%
A typical chromatogramm is shown as Figure 1 (c = 2 μg/mL)

**Example 2:**

The specificity of the lacosamide plasma assay towards other anticonvulsant drugs was tested versus Phenytoin, Carbamazepine, Ethosuximide, Gabapentin, Lacosamide, Lamotrigine, Leviteracetam, Phenobarbital, Pregabaline,

Primidone, Tiagabine, Topiramate, Valproic acid, Zonisamide. The respective drugs were added to samples and measured in the method according to example 1. No interference of these drugs with the measurement of lacosamide or of the reference standard was determined.

**Example 3:**

In a clinical study saliva and plasma samples have been taken in parallel. An excellent correlation between lacosamide concentrations in saliva and plasma was found (Figure 2).

The concentrations of lacosamide can be calculated using the following equations:

Saliva concentration of lacosamide = 0.065 + 1.1697 * plasma concentration of lacosamide

Plasma concentration of lacosamide = 0.855 * saliva concentration of lacosamide

These equations were validated by comparing measured lacosamide plasma concentrations with plasma concentrations predicted from measured saliva concentrations. Figure 3 shows the validation of this model.

**Example 4:**

Based on the determination of the actual lacosamide concentration in plasma ($C_{messured}$) measured at time t (in hours after actual administration) and the comparison with the target concentration, an individual dosing may be adapted according to the following formula

$$dose_new = dose_old \cdot \frac{C_{max,expected} \cdot e^{-0.05 \cdot t}}{C_{measured} \cdot e^{-0.05 \cdot 1h}}$$

The expected $C_{max}$ is usually at about 5, 10, 15, or 20 μg/mL for dose 100, 200, 300, or 400 mg bid.

**Example 4.1**

A patient taking 100 mg twice a day is suffering from seizures. A sample is taken 7 h after the last administration of lacosamide. The measured lacosamide plasma concentration is 1.9 μg/mL. The newly requested Cmax is 5 μg/mL, and the new dose has to be

$$dose_new = 100 \cdot \frac{5 \cdot e^{-0.05 \cdot t}}{1.9 \cdot e^{-0.05 \cdot 1h}} = 195 \text{ mg bid (dose has to be doubled)}$$

**Claims**

1. Method of determining lacosamide in a body fluid sample of a mammal, preferably a human patient, wherein said method comprises the following subsequent steps:

   a) adding to said sample a predefined amount of an internal standard, said standard being a lacosamide homologue differing from the structure of lacosamide only by one or more additional groups selected from methyl, ethyl, propyl, methoxy, ethoxy, and halogen, and having a MW of about 255-320 Da,
   b) separating off at least 80% of the protein content from said sample,
   c) subjecting a predefined amount of the protein diminished sample of step b) to an HPLC analysis under appropriate conditions,
   d) determining the amount of lacosamide and said internal standard via a suitable detection mode, preferably

by UV light, on said HPLC column, and
e) calculating the amount and/or concentration of lacosamide in the sample of the mammal.

**2.** Method according to claim 1, comprising the following subsequent steps:

a) adding to said body fluid sample a predefined amount of an internal standard being a lacosamide homologue having the chemical formula I

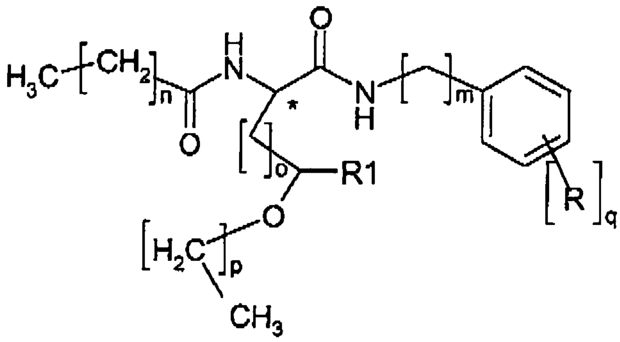

wherein
* indicates a chiral center;
each R is independently selected from methyl, methoxy, fluoro and chloro; R1 is hydrogen or methyl;
m is 1 or 2;
n, o, and p are independently 0 or 1;
q is 0, 1, or 2;
the molecular weight of the internal standard is between about 258 and about 300 Da, preferably of 260-280 Da;
and wherein the internal standard may be in form of a pure enantiomer, diastereomer or any mixture of enantiomers;
b) separating off at least about 90% (w/w) of the protein so as to provide a protein diminished sample
c) analytically separating a pretermined volume of the protein diminished sample of step b) on an HPLC column which has been previously calibrated with said internal standard of formula I,
d) determining the peakheights or peakareas of lacosamide and of the internal standard of formula I after the HPLC separation of step c), and
e) calculating the amount and/or concentration of lacosamide in the body fluid sample using the comparison of the peakheights or peakareas of lacosamide with the respective value of said internal standard of formula I.

**3.** The method of any of the preceding claims, wherein the lacosamide homologue used as internal standard has formula II

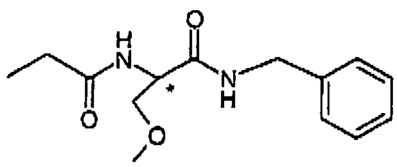

formula II

which may be in form of a pure enantiomer, or any mixture of enantiomers.

**4.** The method of any of the preceding claims, wherein the body fluid sample is blood, serum, plasma or saliva, wherein if the body fluid sample is blood or saliva, the method further comprises the removal of cells by centrifugation prior to step a).

**5.** The method according to anyone of the previous claims wherein protein will be separated off in step c) by adding

a precipitation agent and by subsequently removing the precipitate by centrifugation.

6. The method of claim 5, wherein the precipitation agent is selected from the group of alcohols, acids or organic solvents, preferably from the group of methanol, ethanol, acetonitrile, acetone, trichloroacetic acid (TCA), perchloric acid, acidified methanol, acidified ethanol, acidified acetone, or mixtures thereof.

7. The method of claim 5 according to anyone of the preceding claims, wherein the precipitation agent is methanol, and wherein the volume ratio of methanol: body fluid sample is at least about two.

8. The method of anyone of the preceding claims, wherein the HPLC column is a reversed phase column comprising an organically substituted silane surface, preferably an alkyldimethylsilane surface wherein the alkyl chains each have between 5 and about 20 carbon atoms.

9. The method according to anyone of the preceding claims, wherein the detection of lacosamide during HPLC separation will be performed at about 200-215 nm, preferably at about 205 nm.

10. The method according to anyone of the preceding claims wherein the separation according to step c) of claim 1 will be performed using as the eluent a mixture of an alkali phosphate buffer and acetonitrile at a pH of about 3-5; preferably of about 4-5, and at a flux rate of 0,8-1,5 ml/min.

11. Method of determining the concentration of lacosamide in a patient's blood plasma comprising a method according to anyone of the preceding claims, and subsequently calculating the plasma level of lacosamide based on the lacosamide concentration in the body fluid sample.

12. Kit comprising

(i) an internal standard having the chemical formula I

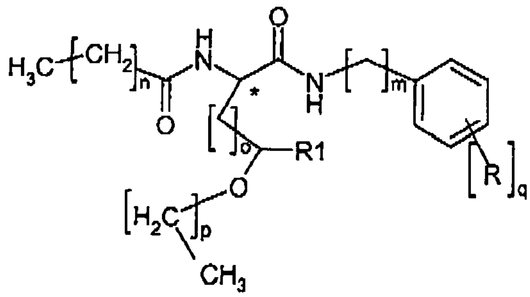

wherein
* indicates a chiral center;
each R is independently selected from methyl, methoxy, fluoro and chloro,
R1 is hydrogen or methyl
m is 1 or 2,
n, o, and p are independently 0 or 1
q is 0, 1, or 2; and
the molecular weight of the internal standard is between about 258 and about 300 Da, preferably of 260-280 Da
and wherein the internal standard may be in form of a pure enantiomer, diastereomer or any mixture of enantiomers,
(ii) instructions for determing the lacosamide concentration in a patients body using the internal standard of formula I, and
(iii) one or more dosing units comprising lacosamide

13. Use of a lacosamide homologue as defined in anyone of claims 1 to 3 as a reference standard in a lacosamide detection assay, preferably for determining the lacosamide concentration or amount in biological samples such as

serum, plasma or saliva.

**14.** Method of determining the concentration of lacosamide in the body of a mammal, particularly of the lacosamide concentration in the blood/plasma/serum of a patient by the steps of

(1) determining the concentration of lacosamide in the saliva sample of a mammal, particularly of a human patient, and
(2) calculating the concentration of lacosamide in the plasma of the mammal, based on the lacosamide concentration determined in step (1)

**Figure 1:** The lacosamide detection assay has been conducted according to Example 1. The peaks at retention times 6,792 and 10.591 min are lacosamide and internal standard respectively, both measured via UV-absorption at a wavelength of 205 nm.

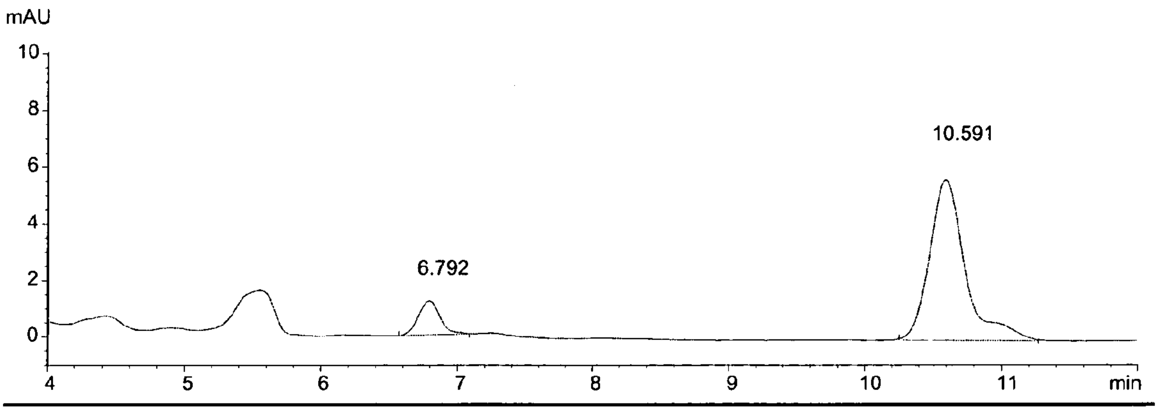

**Figure 2:** Correlation between plasma and saliva concentration of lacosamide

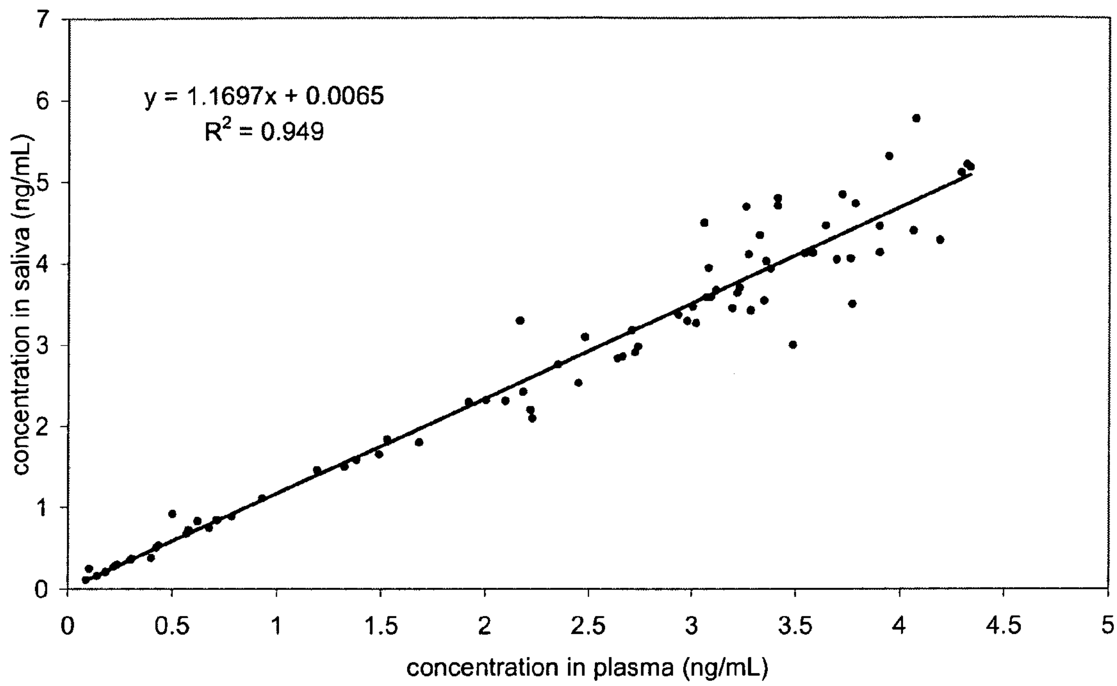

**Figure 3:** Correlation between calculated and measured peak plasma concentrations from a clinical study

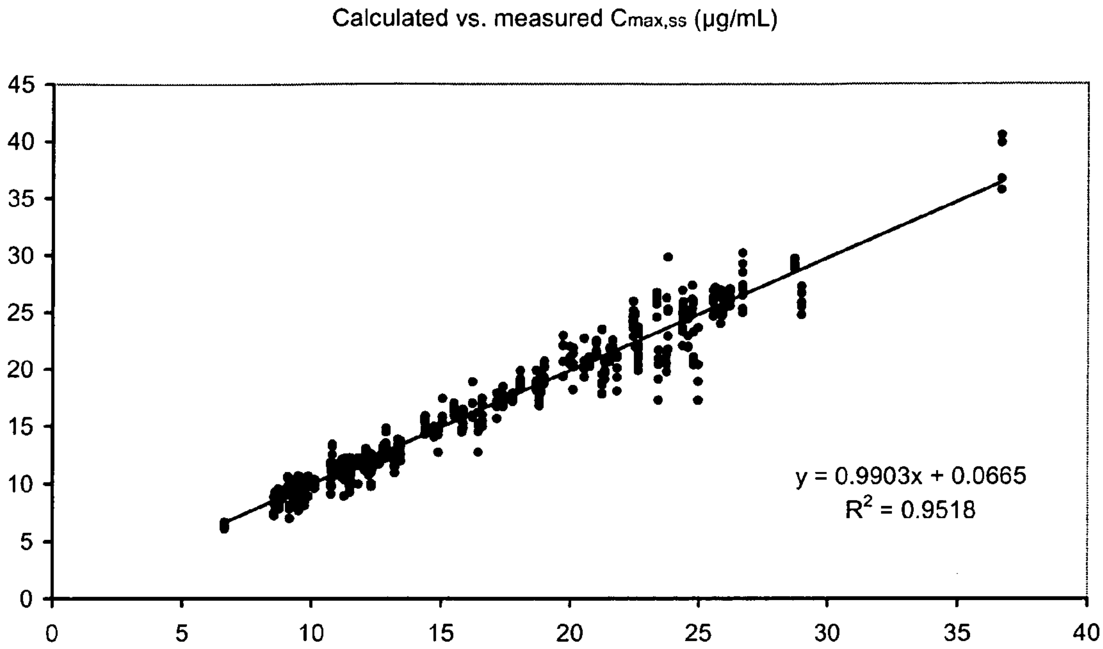

Europäisches Patentamt
European Patent Office
Office européen des brevets

# EUROPEAN SEARCH REPORT

Application Number
EP 10 00 3983

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| A | GREENAWAY C ET AL: "A high-preformance liquid chromatography assay to monitor the new antiepileptic drug lacosamide in patients with epilepsy" THERAPEUTIC DRUG MONITORING, vol. 32, no. 4, 8 April 2010 (2010-04-08), pages 448-452, XP008124456 * the whole document * | 1-14 | INV. G01N33/94 G01N30/00 |
| A | HO CHIH ET AL: "Stability-indicating high-performance liquid chromatographic assay methods for drugs in pharmaceutical dosage forms: Part I" JOURNAL OF FOOD AND DRUG ANALYSIS, vol. 4, no. 4, 1996, pages 271-292, XP002596857 * table 1 * | 1-14 | |
| A | LI J: "Prediction of internal standards in reversed-phase liquid chromatography - 1. Initial study on predicting internal standards for use with neutral samples based on linear solvation energy relationships" JOURNAL OF CHROMATOGRAPHY A, vol. 927, no. 1-2, 2001, pages 19-30, XP004300348 * the whole document * | 1-14 | TECHNICAL FIELDS SEARCHED (IPC): G01N |
| A | LI J: "Prediction of internal standards in reversed-phase liquid chromatography" CHROMATOGRAPHIA, vol. 60, no. 1-2, 2004, pages 63-71, XP002596858 * the whole document * | 1-14 | |

The present search report has been drawn up for all claims

1

| Place of search | Date of completion of the search | Examiner |
|---|---|---|
| The Hague | 17 August 2010 | Gunster, Marco |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P04C01)

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- WO 2007144196 A


### Non-patent literature cited in the description


- **PATSALOS et al.** *Epilepsia,* 2008, vol. 49, 1239-1276